# EUROPEAN PATENT APPLICATION

(11) **EP 3 978 061 A1**
(43) Date of publication of application: **06.04.2022**
(21) Application number: 19930712.5
(22) Date of filing: 28.05.2019
(51) Int. Cl.: A61M 25/10, A61B 17/22

(54) **BALLOON CATHETER**

(71) Applicant: ASAHI INTECC CO., LTD., Seto-shi, Aichi 489-0071 (JP)
(72) Inventor: ITO Naho, Seto-shi, Aichi 489-0071 (JP)
(74) Representative: Winter, Brandl - Partnerschaft mbB
(86) International application number: PCT/JP2019/021162
(87) International publication number: WO 2020/240717

(57) **Abstract**

To provide a balloon catheter capable of preventing debris such as thrombus from adhering to a distal end portion of a hollow shaft and a joint part between a balloon and the hollow shaft.

A balloon catheter 1 includes a hollow shaft 11, and an expandable and contractable balloon 21 placed so as to cover at least a part of an outer periphery of the hollow shaft 11, in which the balloon 21 has a first joint part 21a joined to the outer periphery of the hollow shaft 11, a second joint part 21b located on a proximal end side with respect to the first joint part 21a and joined to the outer periphery of the hollow shaft 11, and an expandable section 21c continuous with the first joint part 21a and the second joint part 21b, the first joint part 21a is wholly located inside the expandable section 21c, and a distal end of the first joint part 21a is spaced apart from a distal end of the hollow shaft 11 toward a proximal end side.

## Description

### TECHNICAL FIELD

The present invention relates to a balloon catheter.

### BACKGROUND ART

When removing thrombus formed in a blood vessel or treating a calcified part (e.g. chronic total occlusion: CTO) in a blood vessel, it is necessary to prevent debris such as the removed thrombus from flowing through a bloodstream and into a peripheral blood vessel. In such a case, a technique is used, in which the debris is intercepted by placing a balloon of a balloon catheter downstream of a treatment part and temporarily blocking the bloodstream.

For such a balloon catheter, in order to smoothly deliver the balloon in a decreased-diameter state to the treatment part, for example, a flexible distal end portion for leading the balloon is disposed on a hollow shaft, in some cases. According to such a catheter, an expandable section of the balloon guided by the distal end portion can be smoothly pushed forward to the treatment part in the blood vessel, and a damage to the blood vessel can be reduced.

### CITATION LIST

### Patent Literature

Patent Literature 1: JP2007-252895

### SUMMARY OF INVENTION

### TECHNICAL PROBLEM

However, in the conventional balloon catheter, the flexible distal end portion is placed on a distal end side with respect to the joint part between the balloon and the hollow shaft, disposed on the distal end of the balloon, and therefore debris such as thrombus adhering to the distal end portion and/or the joint part during treatment may peel off after contraction of the balloon and flow into the peripheral blood vessel.

The present invention has been made on the basis of the above circumstances, and an object of the disclosed embodiments is to provide a balloon catheter capable of preventing debris such as thrombus from adhering to a distal end portion of a hollow shaft and a joint part between a balloon and the hollow shaft.

### SOLUTION TO PROBLEM

This disclosure includes some aspects:
(1) a balloon catheter including
   a hollow shaft and
   an expandable and contractable balloon placed so as to cover at least a part of an outer periphery of the hollow shaft, in which
   the balloon has a first joint part joined to the outer periphery of the hollow shaft, a second joint part located on a proximal end side with respect to the first joint part and joined to the outer periphery of the hollow shaft, and an expandable section continuous with the first joint part and the second joint part,
   the first joint part is wholly located inside the expandable section, and a distal end of the first joint part is spaced apart from a distal end of the hollow shaft toward a proximal end side,
(2) the balloon catheter according to (1), in which a stretchability of a distal end portion of the expandable section during expansion or contraction of the balloon is greater than a stretchability of a proximal end portion of the expandable section located on the proximal end side with respect to the distal end portion,
(3) the balloon catheter according to (1), in which a flexibility of the distal end portion of the expandable section of the balloon is higher than a flexibility of the proximal end portion of the expandable section located on the proximal end side with respect to the distal end portion, and
(4) The balloon catheter according to any one of (1) to (3), in which a hardness of the distal end of the hollow shaft is lower than a hardness of the proximal end of the hollow shaft.

In the present specification, the "distal end side" means a direction along a longitudinal axis direction of the hollow shaft and toward a treatment part. The "proximal end side" means a direction along the longitudinal axis direction of the hollow shaft and opposite to the distal end side. In addition, the "distal end" refers to a distal end portion of any member or part, and the "proximal end" refers to a proximal end portion of any member or part. The "distal end portion" refers to a part that includes a distal end of any member or part and extends from the distal end to a middle of a way toward the proximal end side. The "proximal end portion" refers to a part that includes a proximal end of any member or part and extends from the proximal end to a middle of a way toward the distal end side.

### ADVANTAGEOUS EFFECTS OF INVENTION

The present invention can provide a balloon catheter capable of preventing debris such as thrombus from adhering to a distal end portion of a hollow shaft and a joint part between a balloon and the hollow shaft.

### BRIEF DESCRIPTION OF DRAWINGS

FIG. 1 is a schematic sectional view of a whole of the first embodiment.
FIG. 2 is a schematic sectional view of a part of the first embodiment, illustrating a use state in FIG. 1.
FIG. 3A is a schematic sectional view of a part of the second embodiment, illustrating an example of a hollow shaft.
FIG. 3B is a schematic sectional view of a part of the second embodiment, illustrating an example of the hollow shaft.
FIG. 3C is a schematic sectional view of a part of the second embodiment, illustrating an example of the hollow shaft.
FIG. 3D is a schematic sectional view of a part of the second embodiment, illustrating an example of the hollow shaft.
FIG. 4 is a schematic sectional view of a part of the third embodiment, illustrating an example of a balloon.
FIG. 5 is a schematic sectional view of a part of the fourth embodiment, illustrating an example of the balloon.
FIG. 6 is a schematic sectional view of a part of the fifth embodiment, illustrating an example of the balloon.

### DESCRIPTION OF ENBODIMENTS

The balloon catheter includes a hollow shaft and an expandable and contractable balloon placed so as to cover at least a part of an outer periphery of the hollow shaft, in which the balloon has a first joint part joined to the outer periphery of the hollow shaft, a second joint part located on a proximal end side with respect to the first joint part and joined to an outer periphery of the hollow shaft, and an expandable section continuous with the first joint part and the second joint part, the first joint part is wholly located inside the expandable section, and a distal end of the first joint part is spaced apart from a distal end of the hollow shaft toward a proximal end side.

The first to fifth embodiments of the disclosed embodiments will be explained below with reference to the figures, but the disclosed embodiments are not limited to the embodiments illustrated in the figures. The dimensions of each part illustrated in the figures are intended to facilitate understanding of the implementation and do not correspond to the actual dimensions.

### [First Embodiment]

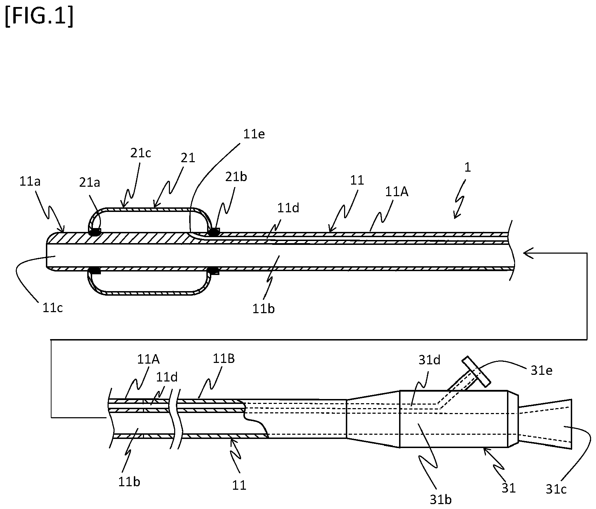

FIG. 1 is a schematic sectional view of a whole of the first embodiment. As illustrated in FIG. 1, a balloon catheter 1 is roughly composed of a hollow shaft 11, a balloon 21, and a connector 31.

The hollow shaft 11 is a shaft having a hollow shape. The hollow shaft 11 has a through-hole (hereinafter also referred to as a "main lumen 11b") having an opening 11c on the distal end and penetrating the hollow shaft 11 from the distal end to the proximal end. For example, a medical device (not illustrated) such as a guide wire and a device for crashing thrombus, or the like is inserted into the main lumen 11b. The hollow shaft 11 has a through-hole (hereinafter also referred to as "expansion lumen 11d") having an opening 11e that adjoins the inside of the balloon 21 described later and penetrating from the opening 11e to the proximal end of the hollow shaft 11. For example, a liquid for expanding and contracting the balloon 21 (hereinafter also referred to as "expansion liquid") passes through the expansion lumen 11d.

The hollow shaft 11 can be composed of e.g. a distal end-side hollow shaft 11A located on the distal end side, and a proximal end-side hollow shaft 11B joined to the proximal end of the distal end-side hollow shaft 11A and extending toward the proximal end side. In the balloon catheter 1, the main lumen 11b and the expansion lumen 11d are provided across the distal end-side hollow shaft 11A and the proximal end-side hollow shaft 11B.

Preferably, materials constituting the distal end-side hollow shaft 11A and the proximal end-side hollow shaft 11B have antithrombogenicity, flexibility and biocompatibility, because the shafts are inserted into a body cavity. For the distal end-side hollow shaft 11A, it is possible to adopt e.g. a resin material such as a polyamide, a polyamide elastomer, a polyolefin, a polyester, a polyester elastomer, a polyurethane, a silicone, and a fluororesin, or the like. For the proximal end-side hollow shaft 11B, it is possible to adopt e.g. a resin material having a higher rigidity than of the distal end-side hollow shaft 11A, a metal material such as a stainless steel (SUS304, and the like) and a superelastic alloy (nickel-titanium alloy, and the like), or the like.

The balloon 21 is an expandable and contractable member placed so as to cover at least a part of the outer periphery of the hollow shaft 11. The balloon 21 has a first joint part 21a, a second joint part 21b, and an expandable section 21c. The expandable section 21c can be expanded by injecting the expansion liquid into the inside of the balloon 21 (a space surrounded by the first joint part 21a, the second joint part 21b, the expandable section 21c, and the outer periphery of the hollow shaft 11) through the opening 11e, so that e.g. the outer peripheral face of the balloon 21 can press an inner wall of a blood vessel or expand a constricted part.

Herein, the joint part between the balloon 21 and the hollow shaft 11 will be explained. The first joint part 21a is a part where the balloon 21 is joined to the outer periphery of the hollow shaft 11. The first joint part 21a is wholly located inside the expandable section 21c. In addition, the distal end of the first joint part 21a is spaced apart from the distal end of the hollow shaft 11 toward the proximal end side. Thus, when the balloon catheter 1 proceeds in the blood vessel, the distal end portion 11a of the hollow shaft leads the balloon 21 in a decreased-diameter state, so that the balloon catheter 1 can be smoothly pushed forward. Specifically, for example, a distal end portion of a sheet-like member constituting the balloon 21 is turned back toward the proximal end side and this turned portion and the outer periphery of the hollow shaft 11 are joined to each other by heat welding or the like, so that the first joint part 21a can be formed.

The second joint part 21b is located on the proximal end side with respect to the first joint part 21a, where the balloon 21 is joined to the outer periphery of the hollow shaft 11. Specifically, for example, after turning back the sheet-like member constituting the balloon 21 toward the proximal end side and joining the sheet-like member at the first joint part 21a, the proximal end portion of the turned portion and the outer periphery of the hollow shaft 11 are joined to each other by heat welding or the like, and thus the second joint part 21b can be formed.

The expandable section 21c is continuous with the first joint part 21a and the second joint part 21b. The expandable section 21c is configured so as to expand radially outward and toward the distal and proximal end sides in the longitudinal axis direction by injecting the expansion liquid into the balloon 21.

The balloon 21 can be formed of e.g. a stretchable sheet-like member. Preferably, a material constituting the balloon 21 has stretchability, ease of joining, antithrombogenicity, and biocompatibility. Examples of such a material include resin materials such as silicone and polyurethane, and the like.

The connector 31 is an operating part where a medical device is inserted or retracted through the main lumen 11b of the hollow shaft 11 and/or where the expansion liquid is injected or removed through the expansion lumen 11d. The connector 31 is connected to the proximal end of the proximal end-side hollow shaft 11B, and includes a passage hole 31b communicating with the main lumen 11b and having an opening 31c on the proximal end side, and a passage hole 31d communicating with the expansion lumen 11d and having an opening 31e on the proximal end side.

In relation to axial-direction lengths of each part in the balloon catheter 1, typically, a whole length of the hollow shaft 11 is 900 mm to 2000 mm, a distance between the distal end of the hollow shaft 11 and the distal end of the first joint part 21a is 1 mm to 10 mm, and a distance between the distal end of the first joint part 21a and the proximal end of the second joint part 21b is 5 mm to 50mm. In relation to outer diameters of each part, typically, an outer diameter of the hollow shaft 11 is 1 mm to 5 mm, and an outer diameter of the balloon 21 is 1 mm to 6 mm in the decreased-diameter state and 2 mm to 20 mm in the increased-diameter state. In relation to an inner diameter of the hollow shaft 11, typically, an inner diameter of the main lumen 11b is 0.5 mm to 3 mm and an inner diameter of the expansion lumen 11d is 0.1 mm to 1mm.

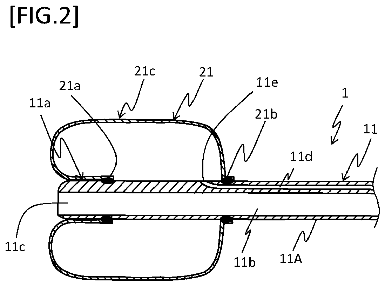

Next, the usage manner of the balloon catheter 1 will be explained with reference to FIG. 1 and FIG. 2. Herein, a procedure in which the balloon catheter 1 is a guiding catheter equipped with a balloon, and thrombus caused in a blood vessel is removed and collected, will be exemplified.

First, prior to insertion of the balloon catheter 1, a guide wire (not illustrated), an angiographic catheter (not illustrated), and the balloon catheter 1 with the balloon 21 in a decreased-diameter state are inserted into a blood vessel. Subsequently, the balloon catheter is pushed forward till just before a part where the thrombus is formed (hereinafter, also referred to as "treatment part") while the guide wire and the angiographic catheter are advanced ahead of the balloon catheter. Subsequently, the guide wire and the angiographic catheter are removed out of the body.

Next, a thrombus collecting device is inserted from the opening 31c of the balloon catheter 1, and a distal end side of the thrombus collecting device is advanced from the opening 11c through the main lumen 11b. Subsequently, the distal end of the thrombus collecting device is pushed forward to the treatment part. Then, an expansion liquid such as physiological saline is injected into the balloon 21 from the opening 31e through the passage hole 31d and the expansion lumen 11d to expand the balloon 21. At this time, in association with injection of the expansion liquid, the outer periphery of the balloon 21 abuts on an inner wall of the blood vessel to intercept the bloodstream, and the balloon 21 proceeds toward the distal end side, so that the outer periphery of the distal end portion 11a of the hollow shaft 11 (outer periphery on the distal end side with respect to the first joint part 21a in the hollow shaft 11) is covered with the expandable section 21c.

Next, the thrombus is crushed, and meanwhile the debris of the thrombus is captured and collected in the thrombus collecting device. Then, after finishing the collection of the thrombus by the thrombus collecting device, the thrombus collecting device and the balloon catheter 1 (in a state that the diameter of the balloon is decreased) are removed out of the body in this order, so that the use of the balloon catheter 1 is completed.

Thus, since the balloon catheter 1 has a configuration in which the whole first joint part 21a is located inside the expandable section 21c, the distal end portion 11a on the distal end side of the hollow shaft 11 can be placed so as to be exposed. The distal end portion 11a of the balloon catheter 1 is made of a soft material that does not damage the blood vessel inner wall even when coming into contact with the blood vessel inner wall. Thus, in the state that the balloon 21 is contracted while inserting the balloon catheter 1 into the body, the distal end portion 11a of the balloon catheter 1 can be exposed. As a result, when inserting the balloon catheter 1 into the blood vessel, damage to the blood vessel inner wall can be suppressed. Furthermore, when the balloon 21 expands, the distal end of the expandable section 21c can proceed to a more distal end side to cover the distal end portion 11a of the hollow shaft 11 and the first joint part 21a, so that debris such as thrombus can be prevented from adhering to the distal end portion 11a and the first joint part 21a. As a result, after the balloon 21 is contracted, the debris such as the thrombus can be prevented from flowing to a peripheral blood vessel.

### [Second Embodiment]

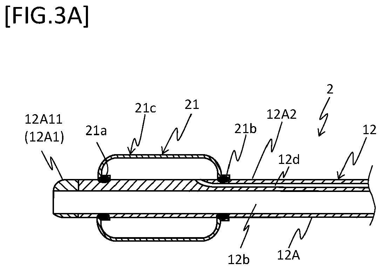

FIG. 3A to FIG. 3D are schematic sectional views of a part of the second embodiment. For example, as illustrated in FIG. 3A, a balloon catheter 2 is roughly composed of a hollow shaft 12, the balloon 21, and the connector 31 (not illustrated). In the balloon catheter 2, the hollow shaft 12 differs from the hollow shaft in the first embodiment. Since the configurations of the balloon 21 and the connector 31 are the same as those in the first embodiment, the same parts are designated by the same reference numerals and detailed description thereof will be omitted. Since configurations other than the following configurations in the hollow shaft 12 and the usage manner of the balloon catheter 2 are the same as those in the first embodiment, detailed description thereof will be omitted.

The hollow shaft 12 has a hollow shape. The hollow shaft 12 can be composed of e.g. a distal end-side hollow shaft 12A located on the distal end side, and a proximal end-side hollow shaft 12B (not illustrated) joined to the proximal end of the distal end-side hollow shaft 12A and extending toward the proximal end side. In the balloon catheter 2, a main lumen 12b and an expansion lumen 12d are provided across the distal end-side hollow shaft 12A and the proximal end-side hollow shaft 12B.

The distal end-side hollow shaft 12A is composed of a distal tip portion 12A1 and a distal end-side hollow shaft body 12A2.

The distal tip portion 12A1 is placed on the distal end portion of the hollow shaft. The distal tip portion 12A1 has a through-hole along the longitudinal axis direction, and this through-hole constitutes a part of the main lumen 12b.

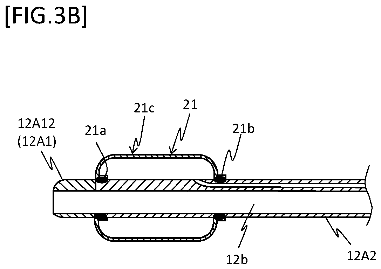

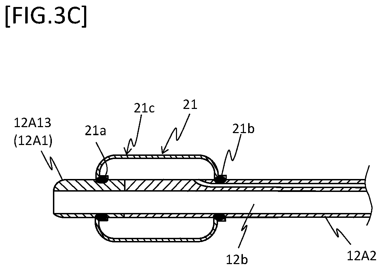

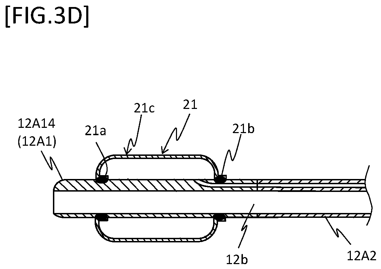

In a part where the distal tip portion 12A1 of the hollow shaft 12 is placed, a proximal end of the distal tip portion 12A1 in the longitudinal direction may be located e.g. on the distal end side with respect to the first joint part 21a (see a distal tip portion 12A11 in FIG. 3A), or located on the same part as the distal end of the first joint part 21a (see a distal tip portion 12A12 in FIG. 3B), or located between the first joint part 21a and the second joint part 21b (see a distal tip portion 12A13 in FIG. 3C), or located on the proximal end side with respect to the second joint part 21b (see a distal tip portion 12A14 in FIG. 3D).

The distal end-side hollow shaft body 12A2 is a part whose distal end is connected to the proximal end of the distal tip portion 12A1. The distal end-side hollow shaft body 12A2 has a through-hole along the longitudinal axis direction, and this through-hole constitutes a part of the main lumen 12b.

Herein, the balloon catheter 2 is formed such that the distal end of the hollow shaft 12 has a hardness lower than of the proximal end of the hollow shaft 12. Specifically, the balloon catheter 2 can be formed e.g. such that the distal tip portion 12A1 has a hardness lower than of the proximal end-side hollow shaft 12B.

As a method for adjusting the hardnesses of the distal end and proximal end of the hollow shaft 12, it is possible to adopt e.g. a method for adjusting the hardness by using the same materials having different structures such as porosity, a method using different materials having different hardnesses, a method of gradually changing the diameter of the hollow shaft, and the like. When using different materials, for example, the materials may be appropriately selected from the materials exemplified as the materials of the hollow shaft 11 in the first embodiment, on the basis of the hardness thereof.

Thus, since the balloon catheter 2 has the aforementioned configuration, the hardness of the distal end is lower than of the proximal end in the hollow shaft 12, so that stimulation to the blood vessel or the like during advancement of the balloon catheter 2 can be reduced.

### [Third Embodiment]

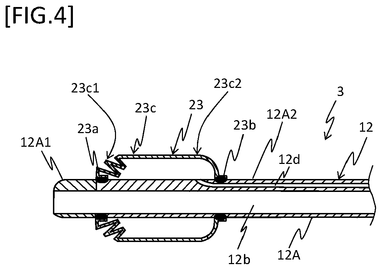

FIG. 4 is a schematic sectional view of a part of the third embodiment. As illustrated in FIG. 4, a balloon catheter 3 is roughly composed of the hollow shaft 12, a balloon 23, and the connector 31 (not illustrated). In the balloon catheter 3, the balloon 23 differs from the balloon in the second embodiment. Since the configurations of the hollow shaft 12 and the connector 31 are the same as those in the second embodiment, the same parts are designated by the same reference numerals and detailed description thereof will be omitted. Since configurations other than the following configurations in the balloon 23 and the usage manner of the balloon catheter 3 are the same as those in the first embodiment, detailed description thereof will be omitted.

The balloon 23 is an expandable and contractable member placed so as to cover at least a part of the outer periphery of the hollow shaft 12. The balloon 23 has a first joint part 23a, a second joint part 23b, and an expandable section 23c continuous with the first joint part 23a and the second joint part 23b.

The first joint part 23a is joined to the outer periphery of the hollow shaft 12. The first joint part 23a is wholly located inside the expandable section 23c. In addition, the distal end of the first joint part 23a is spaced apart from the distal end of the hollow shaft 12 toward the proximal end side. The second joint part 23b is located on the proximal end side with respect to the first joint part 23a, and joined to the outer periphery of the hollow shaft 12. The expandable section 23c is continuous with the first joint part 23a and the second joint part 23b.

The balloon 23 of the balloon catheter 3 is formed such that a stretchability of a distal end portion 23c1 of the expandable section 23c during expansion or contraction of the balloon 23 is greater than a stretchability of a proximal end portion 23c2 of the expandable section 23c located on the proximal end side with respect to the distal end portion 23c1. Specifically, the balloon 23 can be formed e.g. such that the distal end portion 23c1 of the expandable section 23c is folded in a bellows shape in a state where the balloon 23 is contracted (see FIG. 4). When the expansion liquid is injected into the balloon 23, this bellows-like distal end portion 23c1 is expanded so as to be pushed out toward the distal end side.

In this way, since the balloon catheter 3 has the aforementioned configuration, the expandable section 23c can be advanced to a distal end side with respect to the first joint part 23a when expanding the balloon 23. As a result, it is possible to more certainly prevent debris such as thrombus from adhering to the distal end portion of the hollow shaft 12 and the joint part between the balloon 23 and the hollow shaft 12.

### [Fourth Embodiment]

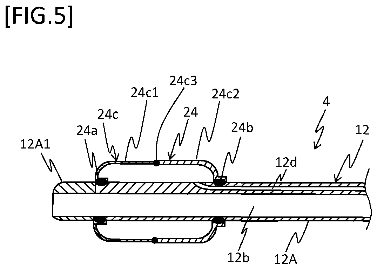

FIG. 5 is a schematic sectional view of a part of the fourth embodiment. As illustrated in FIG. 5, a balloon catheter 4 is roughly composed of the hollow shaft 12, a balloon 24, and the connector 31 (not illustrated). In the balloon catheter 4, the balloon 24 differs from the balloon in the third embodiment. Since the configurations of the hollow shaft 12 and the connector 31 are the same as those in the second embodiment, the same parts are designated by the same reference numerals and detailed description thereof will be omitted. Since configurations other than the following configurations in the balloon 24 and the usage manner of the balloon catheter 4 are the same as those in the first embodiment, detailed description thereof will be omitted.

The balloon 24 is an expandable and contractable member placed so as to cover at least a part of the outer periphery of the hollow shaft 12. The balloon 24 has a first joint part 24a, a second joint part 24b, and an expandable section 24c continuous with the first joint part 24a and the second joint part 24b.

The first joint part 24a is joined to the outer periphery of the hollow shaft 12. The first joint part 24a is wholly located inside the expandable section 24c. In addition, the distal end of the first joint part 24a is spaced apart from the distal end of the hollow shaft 12 toward the proximal end side. The second joint part 24b is located on the proximal end side with respect to the first joint part 24a, and joined to the outer periphery of the hollow shaft 12. The expandable section 24c is continuous with the first joint part 24a and the second joint part 24b.

The balloon 24 of the balloon catheter 4 is formed such that a stretchability of a distal end portion of the expandable section 24c during expansion or contraction of the balloon 24 is greater than a stretchability of a proximal end portion 24c2 of the expandable section 24c located on the proximal end side with respect to a distal end portion 24c1. Specifically, in the balloon 24, for example, the members made of the same materials and having different thicknesses are connected to each other at a connection portion 24c3, and these members can form one expandable section 24c. In the balloon catheter 4, the thinner part 24c1 is placed on the distal end side of the expandable section 24c, and the thicker part 24c2 is placed on the proximal end side of the expandable section 24c. Thereby, the thinner part 24c1 is more stretchable than the thicker part 24c2, and the expandable section 24c is expanded toward a more distal end side when injecting the expansion liquid into the balloon 24.

As a method for connecting the distal end portion 24c1 and the proximal end portion 24c2 to each other in the expandable section 24c, it is possible to adopt e.g. a method of heat-welding resin materials to each other, or the like.

In this way, since the balloon catheter 4 has the aforementioned configuration, the expandable section 24c can be advanced to a distal end side with respect to the first joint part 24a when expanding the balloon 24. As a result, it is possible to more certainly prevent debris such as thrombus from adhering to the distal end portion of the hollow shaft 12 and the joint part between the balloon 24 and the hollow shaft 12.

### [Fifth Embodiment]

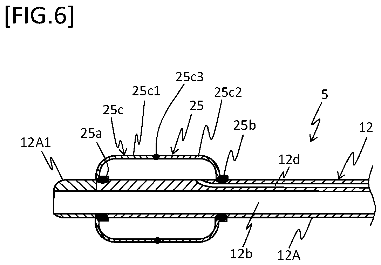

FIG. 6 is a schematic sectional view of a part of the fifth embodiment. As illustrated in FIG. 6, a balloon catheter 5 is roughly composed of the hollow shaft 12, a balloon 25, and the connector 31 (not illustrated). In the balloon catheter 5, the balloon 25 differs from the balloon in the second embodiment. Since the configurations of the hollow shaft 12 and the connector 31 are the same as those in the second embodiment, the same parts are designated by the same reference numerals and detailed description thereof will be omitted. Since configurations other than the following configurations in the balloon 25 and the usage manner of the balloon catheter 5 are the same as those in the first embodiment, detailed description thereof will be omitted.

The balloon 25 is an expandable and contractable member placed so as to cover at least a part of the outer periphery of the hollow shaft 12. The balloon 25 has a first joint part 25a, a second joint part 25b, and an expandable section 25c continuous with the first joint part 25a and the second joint part 25b.

The first joint part 25a is joined to the outer periphery of the hollow shaft 12. The first joint part 25a is wholly located inside the expandable section 25c. In addition, the distal end of the first joint part 25a is spaced apart from the distal end of the hollow shaft 12 toward the proximal end side. The second joint part 25b is located on the proximal end side with respect to the first joint part 25a, and joined to the outer periphery of the hollow shaft 12. The expandable section 25c is continuous with the first joint part 25a and the second joint part 25b.

The balloon 25 of the balloon catheter 5 is formed such that a flexibility of a distal end portion 25c1 in the expandable section 25c of the balloon 25 is greater than a flexibility of a proximal end portion 25c2 in the expandable section 25c located on the proximal end side with respect to the distal end portion 25c1. Specifically, for example, the distal end portion 25c1 and the proximal end portion 25c2 are made of different materials in the expandable section 25c, and are connected to each other at a connection portion 25c3 to form one expandable section 25c, so that the flexibility of the material of the distal end portion 25c1 is greater than the flexibility of the material of the proximal end portion 25c2 (a rigidity of the material of the distal end portion 25c1 is lower than of the material of the proximal end portion 25c2).

A combination of materials used for the distal end portion 25c1 and the proximal end portion 25c2 can be appropriately selected e.g. from the materials constituting the balloon 21 exemplified in the first embodiment on the basis of the flexibility (rigidity).

As a method for connecting the distal end portion 25c1 and the proximal end portion 25c2, it is possible to adopt e.g. the same methods as the methods for connecting the distal end portion 24c1 and the proximal end portion 24c2 of the expandable section 24c aforementioned in the fourth embodiment, or the like.

In this way, since the balloon catheter 5 has the aforementioned configuration, the expandable section 25c can be advanced to a distal end side with respect to the first joint part 25a when expanding the balloon 25. As a result, it is possible to more certainly prevent debris such as thrombus from adhering to the distal end portion of the hollow shaft 12 and the joint part between the balloon 25 and the hollow shaft 12.

As described above, the balloon catheters 1 to 5 make it possible to prevent debris such as thrombus from adhering to the distal end portions of the hollow shafts 11 and 12, and the joint parts 21a to 25a between the balloons 21 to 25 and the hollow shafts 11 and 12. Thus, the balloon catheters 1 to 5 can be suitably applied to e.g. a guiding catheter equipped with a balloon in which a thrombus formed in a blood vessel is removed by passing a thrombus collecting device through the main lumen.

Note that the disclosed embodiments are not limited to the configurations of the aforementioned embodiments, but is stipulated by claims, and the disclosed embodiments are intended to include all modifications within the meaning and scope equivalent to those in claims.

For example, although the balloon catheters 1 to 5 equipped with the single hollow shaft 11 or 12 have been explained in the aforementioned embodiments, the balloon catheter may be a balloon catheter equipped with a plurality of hollow shafts, e.g. a balloon catheter equipped with multiple hollow shafts, including an inner hollow shaft, and an outer hollow shaft placed so as to cover this inner hollow shaft. In such a balloon catheter, for example, a space inside the inner hollow shaft can be used as a main lumen, and a space between the inner hollow shaft and the outer hollow shaft can be used as an expansion lumen.

### REFERENCE SIGNS LIST

- 1 to 5: Balloon catheter
- 11, 12: Hollow shaft
- 21 to 25: Balloon
- 21a to 25a: First joint part
- 21b to 25b: Second joint part
- 21c to 25c: Expandable section
- 23c1, 24c1, 25c1: Distal end portion of expandable section
- 23c2, 24c2, 25c2: Proximal end portion of expandable section

## Claims

1. A balloon catheter comprising:
a hollow shaft; and
an expandable and contractable balloon placed so as to cover at least a part of an outer periphery of the hollow shaft, wherein
the balloon comprises a first joint part joined to the outer periphery of the hollow shaft, a second joint part located on a proximal end side with respect to the first joint part and joined to the outer periphery of the hollow shaft, and an expandable section continuous with the first joint part and the second joint part,
the first joint part is wholly located inside the expandable section, and a distal end of the first joint part is spaced apart from a distal end of the hollow shaft toward a proximal end side.

2. The balloon catheter according to claim 1, wherein a stretchability of a distal end portion of the expandable section during expansion or contraction of the balloon is greater than a stretchability of a proximal end portion of the expandable section located on the proximal end side with respect to the distal end portion.

3. The balloon catheter according to claim 1, wherein a flexibility of the distal end portion of the expandable section of the balloon is higher than a flexibility of the proximal end portion of the expandable section of the balloon located on the proximal end side with respect to the distal end portion.

4. The balloon catheter according to any one of claims 1 to 3, wherein a hardness of the distal end of the hollow shaft is lower than a hardness of the proximal end of the hollow shaft.
